# EUROPEAN PATENT APPLICATION

(11) **EP 4 473 960 A1**
(43) Date of publication of application: **11.12.2024**
(21) Application number: 23177589.1
(22) Date of filing: 06.06.2023
(51) Int. Cl.: A61K 9/00, A61K 39/02, A61K 39/12, A61K 39/00

(54) **VACCINE FORMULATION COMPRISING A SURFACTANT THAT DOES NOT COMPRISE AN ESTER BOND**

(71) Applicant: Vaxxinova Nederland BV, 6534 AT Nijmegen (NL)
(72) Inventor: Mombarg, Erwin, Nijmegen (NL); Janssen, Frank, Nijmegen (NL); Krossøy, Bjørn, Bergen (NO)
(74) Representative: Michalski Hüttermann & Partner Patentanwälte mbB

(57) **Abstract**

The present invention relates to a vaccine formulation comprising at least one inactivated virus or bacterium, or an immunogenic fragment thereof. The formulation further comprises one or more surfactants, wherein at least one surfactant does not comprise an ester bond (Fig. 1).

## Description

### Field of the invention

The present application relates to a Vaccine formulation comprising at least one inactivated virus or bacterium, or an immunogenic fragment thereof.

### Incorporation by Reference

All publications, patents, patent applications and other documents cited in this application are hereby incorporated by reference in their entireties for all purposes to the same extent as if each individual publication, patent, patent application or other document were individually indicated to be incorporated by reference for all purposes. In the event that there are any inconsistencies between the teachings of one or more of the references incorporated herein and the present disclosure, the teachings of the present specification are intended.

### Introduction

A vaccine adjuvant is a substance that increases or modulates the immune response to a vaccine. An immunologic adjuvant is defined as any substance that acts to accelerate, prolong, or enhance antigen-specific immune responses when used in combination with specific vaccine antigens. In the early days of vaccine manufacture, significant variations in the efficacy of different batches of the same vaccine were correctly assumed to be caused by contamination of the reaction vessels. However, it was soon found that more scrupulous cleaning actually seemed to reduce the effectiveness of the vaccines, and some contaminants actually enhanced the immune response.

There are many known adjuvants in widespread use, including aluminium salts, water-in-oils adjuvants and virosomes.

Adjuvants comprising a hydrophobic phase, like e.g. mineral oil, and a surfactant (water-in oil (w/o) adjuvants), are primarily used as animal vaccines. Oftentimes they are used with inactivated (dead) vaccines, to compensate for the somewhat decreased immunogenicity of the inactivated antigen relative to live antigen, as well as for the fact that other than live bacteria or viruses, inactivated bacteria and viruses cannot multiply after vaccination, yet only decrease.

One example is Freund's incomplete adjuvant (IFA), which comprises mineral oil and a surfactant, Arlacel A (Dianhydro-D-mannitol monooleate). Such water-in oil (w/o) adjuvants can trigger depot generation and induction of major histocompatibility complex (MHC) responses. IFA induces a predominantly Th2 biased response with some Th1 cellular response. Oil adjuvants allow the reduction of the vaccine dose or the antigen concentration and can also enhance cellular immune response. However, the precise mode of action of water-in oil (w/o) adjuvants is still unclear. Formulation in emulsions can protect also the antigen from a rapid degradation by enzymes and could modify the electric charge of the antigen becoming more immunogenic. They create an inflammation and stimulate not only the recruitment of antigen presenting cells such as macrophages, but also lymphocytes. They are also able to favour the uptake of antigens by APC and this can be explained by the interactions between the surfactant and the cellular membrane. Lymphocyte trapping is another mechanism of action of oil adjuvants. They stimulate the accumulation of lymphocytes in draining lymph nodes and alter recirculation hence facilitating cell association. Finally, specific cytokines can be induced according to the type of emulsion selected.

Surfactants typically used in vaccine formulations comprising inactivated virus or bacteria have an ester bond, like e.g., Polysorbate 80 (Tween ^{®}), Sorbitan monooleate (Span 80 ^{®}) or (Z)-Dianhydro-D-mannitol monooleate (Arlacel A ^{®})

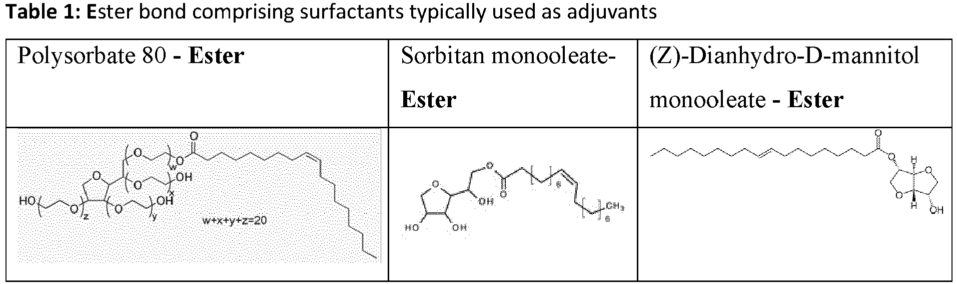

It is desirable for a vaccine, in particular for an animal vaccine, to have a long shelf life even under unfavorable conditions, e.g. when cooling is not always guaranteed throughout the entire delivery and storage chain.

Hence, it is one object of the present invention to provide a vaccine formulation which has a good or improved shelf life even under unfavorable storing conditions.

However, even under favorable storing conditions, shelf life of vaccines may be limited. However, in many cases, e.g., in animal breeding, vaccines are produced and purchased batch wise and then stored for longer periods.

Hence, it is one object of the present invention to provide a vaccine formulation which has a good or improved shelf life even under normal storing conditions.

Furthermore, it is desirable to have a vaccine formulation which has a high protective effect. Hence, it is another object of the present invention top provide a vaccine formulation which has a good or increased protective effect of the vaccine.

### Summary of the invention

These and further objects are met with methods and means according to the independent claims of the present invention. The dependent claims are related to specific embodiments.

### Brief description of the drawings

Figure 1 shows results of a challenge experiment of salmon vaccinated with different formulations and then infected with *A. salmonicida*

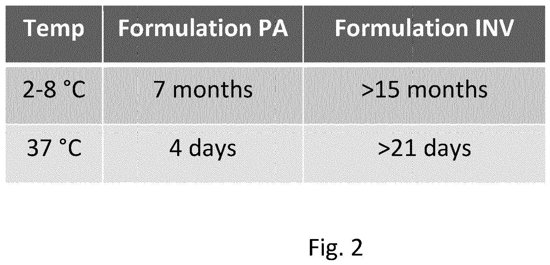

Figure 2 shows results of an accelerated stability study comparing a formulation according to the invention and a formulation according to the state of the art.
Figure 3 shows antibody responses in salmon sera vaccinated with vaccines of different formulations (wpv = weeks post vaccination)
Figure 4 shows antibody titres (log2) (Fig. 4A) and side effect score in pooled sera of salmon vaccinated with different formulations (Fig. 4B)
Figure 5 shows antibody responses against *A.salmonicida* in pooled sera of salmon vaccinated with different formulations

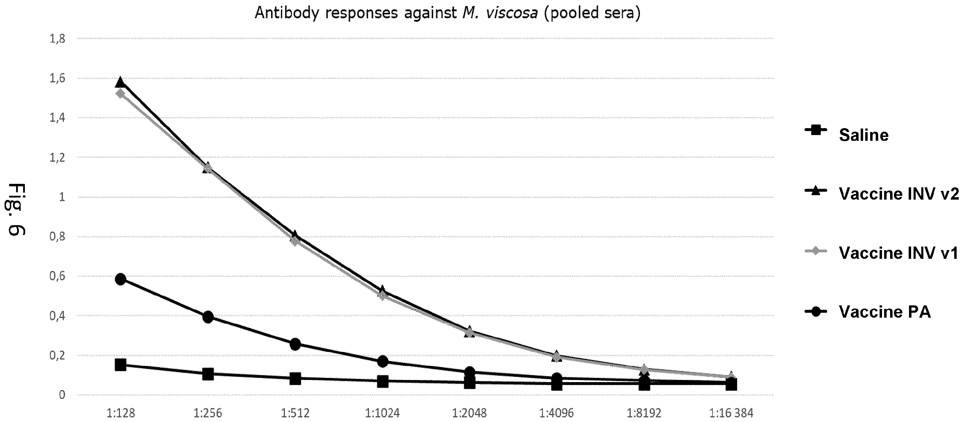

Figure 6 shows antibody responses against *M. viscosa* in pooled sera of salmon vaccinated with different formulations

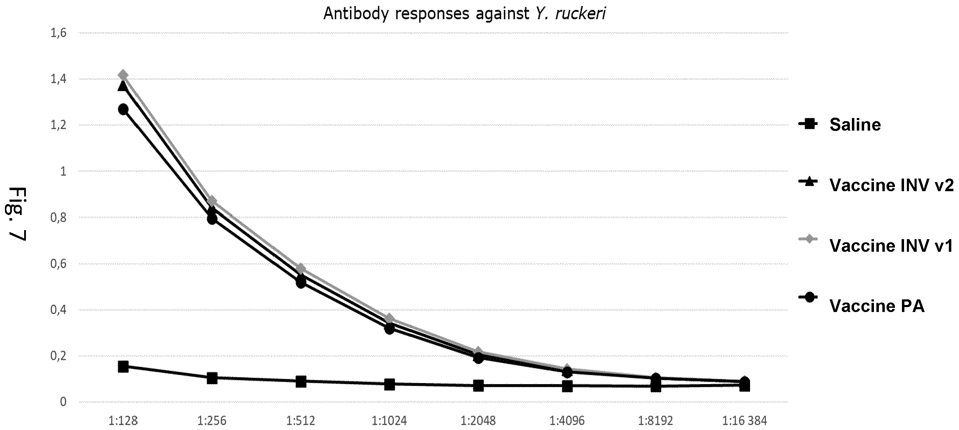

Figure 7 shows antibody responses against *Y. ruckeri* in pooled sera of salmon vaccinated with different formulations

### Embodiments of the invention

Before the invention is described in detail, it is to be understood that this invention is not limited to the particular component parts or structural features of the devices or compositions described or process steps of the methods described as such devices and methods may vary. It is also to be understood that the terminology used herein is for purposes of describing particular embodiments only, and is not intended to be limiting. The mere fact that certain measures are recited in mutually different dependent claims does not indicate that a combination of these measures cannot be used to advantage. Any reference signs in the claims should not be construed as limiting the scope. It must be noted that, as used in the specification and the appended claims, the singular forms "a," "an" and "the" include singular and/or plural referents unless the context clearly dictates otherwise. Further, in the claims, the word "comprising" does not exclude other elements or steps.

It is moreover to be understood that, in case parameter ranges are given which are delimited by numeric values, the ranges are deemed to include these limitation values.

It is further to be understood that embodiments disclosed herein are not meant to be understood as individual embodiments which would not relate to one another. Features discussed with one embodiment are meant to be disclosed also in connection with other embodiments shown herein. If, in one case, a specific feature is not disclosed with one embodiment, but with another, the skilled person would understand that does not necessarily mean that said feature is not meant to be disclosed with said other embodiment. The skilled person would understand that it is the gist of this application to disclose said feature also for the other embodiment, but that just for purposes of clarity and to keep the specification in a manageable volume this has not been done.

According to one aspect of the invention, a vaccine formulation comprising at least one inactivated virus or bacterium, or an immunogenic fragment thereof, is provided. The formulation further comprising one or more surfactants, wherein at least one surfactant does not comprise an ester bond.

The inventors have shown that vaccine formulations comprising such ester-bond containing surfactants may have stability issues. These stability issues can be overcome in case a surfactant is used that does not comprise an ester bond.

The inventors have surprisingly shown that formulations comprising a surfactant that does not comprise an ester bond have an improved shelf life Furthermore, they have shown that such formulations have an improved protective effect.

Without being bound to theory, one explanation could be that such formulations are less susceptible to lipases comprised in the vaccine. Such lipases catalyze the hydrolysis of ester bonds, and as such may be responsible for the degradation of surfactants in the vaccine formulation which comprise such ester bonds, thereby affecting the stability of the formulation, leading to phase separation of the water-in oil (w/o) emulsion.

Such lipases can either be present in the formulation as a result of impurities or contaminations, yet also can be released by the pathogens in the formulation - even if the pathogens are inactivated or dead.

Without being bound to theory, the improved protective effect demonstrated by the inventors can be due to said improved stability ("indirect effect"). However, it appears that a surfactant that does not comprise an ester bond even as such improves the adjuvant efficacy of the water-in oil (w/o) emulsion ("direct effect").

According to one embodiment of the invention, the surfactant that does not comprise an ester bond is a Polyalkylenglycolether or a Polyoxyethylenoleylether.

Polyalkylenglycolethers are non-ionic surfactants, The lipophilic part of these surfactants consists of fatty alcohols, while the hydrophilic part forms polyethylene glycols (polyoxyethylenes). The fatty alcohols used are those derived, *inter alia*, from lauric, palmitic, stearic or oleic acid. Being ethers, they do not comprise an ester bond.

According to one embodiment of the invention, the surfactant that does not comprise an ester bond have is selected from the group consisting of Brij (Croda International Pic), Genapol (Clariant) and Lutensol (BASF),

According to one embodiment of the invention, the surfactant that does not comprise an ester bond have is Brij^{®}O20. Brij^{®} O20 (CAS identifier: 9004-98-2, sum formula: C58H116O21/ C₁₈H₃₅(OCH₂CH₂)ₙOH, n~20) is a non-ionic surfactant that has an 18 carbon tail with a single double bond and the head contains 20 ethylene oxide moieties. It is a polyethoxylated fatty alcohol that is hydrophilic in nature.

According to other embodiments of the invention, the surfactant that does not comprise an ester is a surfactant shown in table 2.

**Table 2: Examples of surfactants that can be used in vaccine formulations according to the invention**

| **group** | **chemical designation** | **other names** | **g/mol** | **HLB** | **Trade** name |
|---|---|---|---|---|---|
| Polyoxyethylenether of Lauryl alcohols (12 C atoms), CAS#9002-92-0, Macrogollaurylether | Polyoxyethylen (4) laurylether | Laureth-4 (INCI) | 363 | 9,7 | Brij 30 |
| | Polyoxyethylen (9) laurylether | Laureth-9 (INCI) | 583 | 11.2 | Thesit |
| | Polyoxyethylen (23) laurylether | Laureth-23 (INCI) | 1199 | 16,9 | Brij 35 |
| Polyoxyethylenether of Cetyl alcohols (16 C atoms), CAS#9004-95-9 | Polyoxyethylen (2) cetylether | Ceteth-2 (INCI) | 1123,5 | 5,3 | Brij 52 |
| | Polyoxyethylen (10) cetylether | Ceteth-10 (INCI) | 1123,5 | 12,9 | Brij 56 |
| | Polyoxyethylen (20) cetylether | Ceteth-20 (INCI) | 1123,5 | 15,7 | Brij 58 |
| Polyoxyethylenether of Cetylstearyl alcohols (16/18 C atoms), CAS#68439-49-6, Macrogolcetyl-stearylether | Polyoxyethylen (6) cetylstearylether | Ceteareth-6 (INCI) | 534 | 10-12 | Cremoph or A6 |
| | Polyoxyethylen (20) cetylstearylether | Ceteareth-20 (INCI) | 1150 | 14-16 | Cremoph or A20 |
| | Polyoxyethylen (25) cetylstearylether | Ceteareth-25 (INCI) | 1370 | 15-17 | Cremoph or A25 |
| Polyoxyethylenether of Stearyl alcohols (18 C atoms), CAS#9005-00-9, Macrogolstearylethe r | Polyoxyethylen (2) stearylether | Steareth-2 (INCI) | 1151,56 | 4,9 | Brij 72 |
| | Polyoxyethylen (10) stearylether | Steareth-10 (INCI) | 1151,56 | 12,4 | Brij 76 |
| | Polyoxyethylen (20) stearylether | Steareth-20 (INCI) | 1151,56 | 15,3 | Brij 78 |
| Polyoxyethylenether of Oleyl alcohols (18 C atoms), CAS#9004-98-2, Macrogololeylether | Polyoxyethylen (2) oleylether | Oleth-2 (INCI) | 1149,53 | 4,9 | Brij 92 |
| | Polyoxyethylen (10) oleylether | Oleth-10 (INCI) | 1149,53 | 12,4 | Brij 96, Brij 97 |
| | Polyoxyethylen (20) oleylether | Oleth-20 (INCI) | 1149,53 | 15,3 | Brij 98, BrijO20 |
| other Polyalkylenglycoleth er | Polyoxyethylen (10) monodecylether ( | Deceth-10 (INCI) | 598.86 | 14.6 | |
| | Polyoxyethylen (10) tridecylether (CAS#24938-91-8) | Trideceth-10 (INCI) | 640.56 | 13.7 | |
| poly(ethylene oxide) (PEO) polypropylene oxide) (PPO) block polymers | Non-ionic copolymer surfactant | | 12600 | 22 | Pluronic F127 |

According to one embodiment, the surfactant that does not comprise an ester bond has a molecular weight of ≥ 600 g/mol.

In embodiments, the surfactant that does not comprise an ester bond has a molecular weight of ≥ 650 g/mol, ≥ 700 g/mol, ≥ 750 g/mol, ≥ 800 g/mol, ≥ 850 g/mol, ≥ 900 g/mol, ≥ 950 g/mol, ≥ 1000 g/mol, ≥ 1050 g/mol or ≥ 1100 g/mol.

According to embodiments of the invention, the formulation further comprises at least one hydrophobic ingredient selected from the group consisting of
- lipid
- natural oil,
- hydrogenated natural oil
- mineral oil
- branched hydrocarbon and/or
- unbranched hydrocarbon

The term "lipid" as used herein encompasses any known lipid. In particular, the term, as used herein, comprises saturated fatty acids, unsaturated fatty acids, glycerolipids, glycerophospholipids, lysophosphoglycerolipids, sphingolipids, sterol lipids, prenol lipids, saccharolipids, carotenoids, waxes, and polyketides.

As used herein, the term "natural oil" may refer to oil derived from plants or animal sources. The term "natural oil" includes natural oil derivatives, unless otherwise indicated. Examples of natural oils include, but are not limited to, vegetable oils, algae oils, animal fats, tall oils, derivatives of these oils, combinations of any of these oils, and the like. Representative nonlimiting examples of vegetable oils include canola oil, rapeseed oil, coconut oil, corn oil, cottonseed oil, olive oil, palm oil, peanut oil, safflower oil, sesame oil, soybean oil, sunflower oil, linseed oil, palm kernel oil, tung oil, j atropha oil, mustard oil, camelina oil, penny cress oil, hemp oil, algal oil, jojoba oil, and castor oil.

As used herein, the term "hydrogenated natural oil" refers to partial, complete, or substantially complete hydrogenation of a natural oil. Partial or substantially complete hydrogenation of natural oils is well known in the art and many hydrogenated natural oils may be purchased on the market and are available from a variety of commercial sources.

The term "mineral oil" refers to a mixture of liquid hydrocarbons obtained from petroleum, coal, shale, or synthezised by Fischer-Tropsch synthesis, as distinguished from an oil of direct vegetable or animal origin.

According to one embodiment of the invention, the surfactant and the lipid or oil act as an adjuvant.

Furthermore, the inventors observed, surprisingly, that a vaccine formulated according to the present invention has better protective effect on vaccinated subjects than a vaccine formulated according to the state of the art, comprising surfactants which all have an ester bond (like e.g. polysorbate 80 (a.k.a. Polyoxyethylene (80) sorbitan monooleate, Tween 80 ^{®}) as a surfactant. This may, in part, be due to the better stability of the vaccine formulation according to the invention (Woodard LF, Jasman RL. Stable oil-in-water emulsions: preparation and use as vaccine vehicles for lipophilic adjuvants. Vaccine. 1985 Jun;3(2): 137-44).

According to embodiments of the invention, the vaccine is polyvalent and comprises two or more inactivated virus or bacteria, or immunogenic fragments thereof.

According to embodiments of the invention, the virus or bacteria have been inactivated by application of at least one of
- formaldehyde
- heat,
- radiation
- ethyleneimine and/or
- beta-propiolactone (BPL)

According to one embodiment of the invention, at least one bacterium in the formulation is a bacterium that produces, secretes and/or releases a lipase.

According to one embodiment of the invention, the lipase is a heat stable lipase. In the meaning of the present invention, the term "heat stable lipase" relates to a lipase that retains > 50 % activity after exposure to at least 60 °C for one hour.

In one embodiment, the vaccine comprises, *inter alia*, inactivated *Yersinia ruckeri*, *Aeromonas salmonicida* and *Moritella viscosa.* The inventors have found that *Aeromonas salmonicida* and *Moritella viscosa* comprise lipases that are capable to break down ester bonds as for example in Tween 80 or Span 80.

The inventors have further shown that thermal inactivation at between 60 °C for 70 hrs and 80 °C for 1 hr deactivates the lipase in *Moritella viscosa*, without affecting the immunogenicity of its antigens. In contrast thereto, *Aeromonas salmonicida,* must be inactivated under milder conditions in order to not affect the immunogenicity of its antigens. Without being bound to theory, however, it appears that such mild conditions are insufficient to deactivate its lipase.

According to one embodiment of the invention, the vaccine formulation comprises one or more further surfactants.

In one embodiment, one such further surfactant is a surfactant that comprises an ester bond. In one embodiment, one such further surfactant is Sorbitan monooleate (Span 80^{®}).

In other embodiments, at least one further surfactant that comprises an ester bond can be selected from the group consisting of Sorbitan laureate (Span 20^{®}), Sorbitan palmitate (Span 40^{®}), Sorbitan stearate (Span 60^{®}), Sorbitan tristearate (Span 65^{®}) or Sorbitan trioleate (Span 85^{®}).

Hence, though one key feature of the formulation according to the invention is that is comprises at least one surfactant does not comprise an ester bond, the formulation may, in addition, comprise further surfactants that comprise ester bonds.

Without being bound to theory, the inventors have noted that the effect caused by the presence of the least one surfactant which does not comprise an ester bond is not impaired by further surfactants that comprise ester bonds.

In one embodiment, the surfactant that comprises an ester bond has a Hydrophilic-lipophilic balance (HLB) of ≤ 10.

Hydrophilic-lipophilic balance (HLB) is the balance of the size and strength of the hydrophilic and lipophilic moieties of a surfactant molecule. The HLB scale ranges from 0 to 20. In the range of < 3,5, surfactants are mainly lipid soluble. In the range of 3.5 to 10, surfactants are more suitable for use in W/O emulsions, where they mainly concentrate in the lipid phase. Surfactants with HLB values in the 10 to 18 range are most commonly used in O/W emulsions, where they mainly concentrate in the aqueous phase.

Without being bound to theory, it could be that, because lipases are enzymes that are mainly hydrophilic, their detrimental effect on ester bond-comprising surfactants applies primarily to surfactants that mainly concentrate in the aqueous phase, i.e. surfactants with an HLB of > 8. For this reason, in vaccine formulation comprising water and oils, Polysorbate 80 may become subject to lipase digestion, while Sorbitan Oleate may be protected from such digestion.

In some embodiments, at least one surfactant that comprises an ester bond has a Hydrophilic-Lipophilic Balance (HLB) of ≤ 9, ≤ 8, ≤ 7, ≤ 6, or ≤ 5.

**Table 3A: HLB values of surfactants discussed herein**

| Surfactant | Alias | Ester bond ? | HLB |
|---|---|---|---|
| Polysorbate 80 | Tween 80 | Yes | 15 |
| Sorbitan laureate | Span 20 | Yes | 8,6 |
| Sorbitan palmitate | Span 40 | Yes | 6.7 |
| Sorbitan stearate | Span 60 | Yes | 4,7 |
| Sorbitan monooleate | Span 80 | Yes | 4,3 |
| Sorbitan tristearate | Span 65 | Yes | 2,2 |
| Sorbitan trioleate | Span 85 | Yes | 1,8 |
| Polyoxyethylenlaurylether / Macrogollaurylether | Brij 30 | No | 9,7 |
| Polyoxyethylenlaurylether | Brij 35 | No | 16,9 |
| Polyoxyethylencetylether | Brij 52 | No | 5,3 |
| Polyoxyethylencetylether | Brij 56 | No | 12,9 |
| Polyoxyethylencetylether | Brij 58 | No | 15,7 |
| Polyoxyethylenstearylether | Brij 72 | No | 4,9 |
| Polyoxyethylenstearylether | Brij 76 | No | 12,4 |
| Polyoxyethylenstearylether | Brij 78 | No | 15,3 |
| Polyoxyethylenoleylether / Macrogololeylether | Brij 92 | No | 4,9 |
| Polyoxyethylenoleylether | Brij 96 | No | 12,4 |
| Polyoxyethylenoleylether | BrijO20 | No | 15,3 |
| Polyoxyethylenstearylether / Macrogol-6-cetylstearylether | Cremophor A6 | No | 10 - 12 |
| Polyoxyethylenstearylether / Macrogol-25-cetylstearylether | Cremophor A25 | No | 15-17 |

According to embodiments of the invention, the surfactant that does not comprise an ester bond has a Hydrophilic-Lipophilic Balance (HLB) of ≥ 8, ≥ 9, ≥ 10, ≥ 11, ≥ 12, ≥ 13, ≥ 14, or ≥ 15.

According to embodiments of the invention, in which the formulation comprises two or more surfactants, the overall Hydrophilic-Lipophilic Balance (HLB) of the formulation is ≥ 6. This means, for example, if the additional surfactant that comprises an ester bond has a Hydrophilic-Lipophilic Balance (HLB) of for example 4,3 (like e.g. Sorbitan monooleate (Span 80), the surfactant that does not comprise an ester bond needs to have a higher HLB to compensate for the said low HLB.

This means, for example, that in a formulation comprising e,g, 43 grams of a first surfactant having a HLB of 4,3 and 11 grams of a second surfactant, the HLB of the second surfactant must be 15,30 or higher. See the following table for a model calculation:

**Table 3B: example of mixtures of different surfactants and resulting HLB**

| surfactant No | grams | HLB | concentration compensated HLB |
|---|---|---|---|
| 1 | 43 | 4,3 | 3,4 |
| 2 | 11 | 15,30 | 3,1 |
| **Total** | **54** | **6,5** | |

According to another aspect of the invention, a vaccine comprising the vaccine formulation according to any one of the aforementioned claims is provided.

According to another aspect of the invention, use of the vaccine formulation according to any one of the aforementioned claims for the manufacture of a vaccine is provided.

According to another aspect of the invention, a formulation according any one of the aforementioned claims (for the manufacture of a vaccine) for the treatment of an animal or human is provided.

This language is deemed to encompass both the swiss type claim language accepted in some countries (in this case, brackets are deemed absent) and EPC2000 language (in this case, brackets and content within the brackets is deemed absent).

According to embodiments of the invention, the animal is at least one selected from the group consisting of bird, mammals including ruminants, non-ruminants, and ungulates, and fish.

According to embodiments of the invention, the fish is a salmonid. In one embodiment, the fish is salmon (*Salmo salar*).

### Examples

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

### Materials and Methods

The 6 bacterial antigens for a fish vaccine were produced in house. The PBS was purchased at Dulbecco. Polyoxyethylene (80) sorbitan monooleate and Sorbitane monooleate were provided by Seppic. Polyoxyethylene (20) oleyl ether was provided by Croda and the Marcol 52 was purchased at Exxon.

The 0.2 µm PES filters used for the filtration of the oil phases were purchased at Sartorius. The emulsification equipment was an ultra turrax of the IKA company and the N18 rotor stator was used.

### Production of the vaccine

The vaccine is a sterile water-in-oil formulation. The sterile formaldehyde inactivated pathogens are in the dispersed water phase. The composition of the antigen phase is in Table 4:

**Table 4: composition of the dispersed antigen phase.**

| Antigen phase | For 1 liter vaccine |
|---|---|
| *Aeromonas salmonicida 1* | 28,87g |
| *Listonella anguillarum Serotyp O1* | 53,21g |
| *Listonella anguillarum serotype O2* | 33,53g |
| *Vibrio salmonicida* | 21,16g |
| *Moritella viscosa* | 26,46g |
| *Yersinia ruckeri serotype O1* | 4,06g |
| PBS | 282,71g |
| | |
| Total | 450g |

In the prior art formulations ("formulation PA"), the antigen phase is dispersed in an oil phase with the following compositions (antigen phase as in table 4, product called "vaccine PA" then):

**Table 5: Composition of the oil phase of a formulation according to prior art ("formulation PA")**

| **Continuous oil phase** | **Supplier** | **Amount** | **Dimension** |
|---|---|---|---|
| Polyethylene sorbitan monooleate (Polysorbate 80) | Seppic | 11 | grams |
| Sorbitane monooleate (Span 80) | Seppic | 43 | grams |
| Mineral oil (Marcol 52) | Exxon | 412 | grams |
| | | | |
| Total | | 467 | grams |
| Total | | 550 | ml |

In a formulation according to the invention ("formulation INV"), Polyethylene sorbitan monooleate (Polysorbate 80, Tween 80 ^{®}) was replaced 1:1 by a surfactant that does not comprise an ester bond, e.g. Polyoxyethylene (20) oleyl ether (BrijO20 ^{®}). The product has therefore the following oil phase (for the complete vaccine, antigen phase was used as in table 4, product called "vaccine INV" then):

**Table 6: composition of the oil phase of a formulation according to the invention ("formulation INV"**

| **Continuous oil phase** | **Supplier** | **Amount** | **Dimension** |
|---|---|---|---|
| Polyoxyethylene (20) oleyl ether (BrijO20) | Croda | 11 | grams |
| Sorbitane monooleate (Span 80) | Seppic | 43 | grams |
| Mineral oil (Marcol 52) | Exxon | * 411 | grams |
| | | | |
| Total | | 466 | grams |
| Total | | 550 | ml |

| | | | |
|---|---|---|---|
| * correction for the lower density of Polyoxyethylene (20) oleyl ether (BrijO20) compared to Polyoxyethylene (80) sorbitan monooleate to keep the amount of injected surfactants per 0.05 ml injection volume constant. | | | |

The oil phases were filtrated over a sterile 0.2 µm PES filter.

The following table shows the composition of a vaccine according to the prior art which comprises exclusively surfactants with ester bonds.

**Table 7: composition of the oil phase of a competing vaccine formulation ("Vaccine COMP")**

| **Continuous oil phase** | | **Dimension** |
|---|---|---|
| Polyethylene sorbitan monooleate (Polysorbate 80) | | grams |
| Sorbitane monooleate (Span 80) | | grams |
| Mineral oil (Marcol 52) | | grams |
| **Antigen phase** | | |
| *Aeromonas salmonicida* subsp. *salmonicida* | RPS ≥ 70 % | |
| *Listonella anguillarum* serotype O1 | RPS ≥ 75 % | |
| *Listonella anguillarum*^{∗} serotype O2a | RPS ≥ 75 % | |
| *Vibrio salmonicida* | RPS ≥ 90 % | |
| | | |
| *Moritella viscosa* | RPS ≥ 60 % | |
| Infectious Pancreatic Necrosis Virus serotype Sp | 0.12-0.28 AU3 | |

| | | |
|---|---|---|
| ^{∗}RPS = relative percentage survival ^{∗} AU = Antigenicity Units | | |

The temperature of the oil phase and antigen phase were 25 °C the moment they are mixed. A magnetic stirring bar was added aseptically to the oil phase and during the hole procedure the magnetic stirring speed was 300 rpm. The sterile IKA ultra turrax rotor stator, set at 8000 rpm, was also in the oil phase. The scale of production was 250 ml. The procedure started with the slow addition of the antigen phase to the magnetically stirred oil phase. When the antigen phase was completely added, the ultra turrax was started. When emulsion was mixed for 4 min at 8000 rpm turrax speed, the turrax was stopped. The product was filled in glass vials and stoppered with nitril rubber stoppers and capped. The products were stored at 5 °C till use.

The same approach was used for a viral chicken vaccine. The viral antigens are also produced in house.

### Adherence score

The adherence score is based on a system developed for evaluating side effects in fish following vaccination of Atlantic salmon with oil-based vaccines. The vaccines are deposited in the abdominal cavity and the scoring is based on autopsy of fish post vaccination. The following table providing Speilberg score gradings (Midtlyng PJ (1996) A field study on intraperitoneal vaccination of Atlantic salmon (Salmo salar L.) against furunculosis. Fish Shellfish Immunol 6:553-565) is used:

**Table 8: Speilberg score gradings**

| Score | Visual appearance of abdominal cavity | Severity of lesion |
|---|---|---|
| 0 | No visual lesions | None |
| 1 | Very slight adhesions most frequently localised close to the injection site. Unlikely to be noticed by laymen during evisceration | No or minor opacity of peritoneum after evisceration |
| 2 | Minor adhesions, which may connect colon, spleen or caudal pyloric caeca to the abdominal wall. May be noticed by laymen during evisceration. | Only opacity of peritoneum remaining after manually disconnecting the adhesions. |
| 3 | Moderate adhesions including more cranial parts of the abdominal cavity, partly involving pyloric caeca, the liver or ventricle, connecting them to the abdominal wall. May be noticed by laymen during evisceration. | Minor visible lesions after evisceration, which may be removed manually. |
| 4 | Major adhesions with granuloma, extensively interconnecting Internal organs, which thereby appear as one unit. likely to be noticed by laymen during evisceration. | Moderate lesions, which may be hard to remove manually. |
| 5 | Extensive lesions affecting nearly every Internal organ in the abdominal cavity. In large areas, the peritoneum is thickened and opaque, and the fillet may carry focal, prominent and/or heavily pigmented lesions or granulomas. | Leaving visible damage to the carcass after evisceration and removal of lesions. |
| 6 | Even more pronounced than 5, often with considerable amounts of melanin. Viscera cannot be removed without damage to fillet integrity. | Leaving major damage to the carcass. |

Typically 30 fish are evaluated at different timepoints after vaccination, and each fish is given a certain score based on the objective impression of the veterinary/ fish health biologist performing the autopsy. An average score of between 2 and 3 is normally regarded as acceptable, unless there is a huge spread in the results and there are a lot of score 3 fish.

### Antibody titre

The antibody titre is reflecting the dilution at which the titration curve crosses a background level specific for the assay.

For example, for *Y. ruckeri the* background level is calculated as the mean of 8 negative control samples multiplied by a factor of 1.47 (the factor is decided in a validation study). At the point where the titration curve crosses the background level, the dilution is determining the titre. The dilution typically starts at 1:128, and from there on two-folds dilutions (log2 of 128= 7). If the log2 titre is 14 this means that the crossing happens at dilution 1: 16384.

Alternatively, the whole titration curve is shown, as in most of the graphs. The y-axis then represents the spectrophotometric reading (optical density) of the colour reaction at 450 nm.

### Stability studies

Accelerated stability studies were performed in tubes stored at 37 °C. It was monitored whether the emulsion is still homogeneous. White or large (transparent) droplets of dispersed phase could be observed on the bottom of the tube with the naked eye (broken emulsion). Real time stability studies was performed in the same way, but the storage temperature was 2-8 °C.

### Test experiment

The same approach was be used for a viral chicken vaccine. The viral antigens were also produced in house.

Monovalent vaccines based on inactivated IBV (Infectious Bronchitis Virus in chicken) and NDV (Newcastle Disease Virus) were produced. The prior art vaccine with Polysorbate 80, Sorbitane monooleate and mineral oil was made with 4 different concentrations (50%, 40%, 30% and 15%) IBV or (30, 22.5, 15 and 7.5%) NDV inactivated antigens.

For a formulation according to the invention a formulation with Polyoxyethylene (20) oleyl ether, Sorbitane monooleate and mineral oil was produced with 30% and 15% IBV or 22,5% and 15% NDV inactivated antigens. With this set-up the potential positive effect of Polyoxyethylene (20) oleyl ether could be demonstrated by obtaining equivalent or higher antibody titers with a lower percentage antigen in the vaccine.

### Example 1: In vivo testing of formulations

The experiment contained the following test vaccines and controls:

**Table 9: test vaccines and controls:**

| **Formulation no.** | **Treatment** | **Batch no.** | **Labelling** |
|---|---|---|---|
| 1 | Vaccine PA | FAO-21-NED-141.20 | ANT-Red |
| 2 | Vaccine INV v1 | FAO-21-NED-142.6 | ANT-Red + RM |
| 2 | Vaccine INV v2 | FAO-22-NED-162.1 | RM+AF |
| 10 | Control | Saline | LM + AF |

The experiment has the following timeline of the *in vivo* part of the experiment:

**Table 10: timeline of the in vivo part of the experiment**

| **Event no.** | **Project week (PW)** | **Calendar week** | **Task** |
|---|---|---|---|
| 1 | -2 | 46-2021 | Receival of test vaccines |
| 2 | 0 | 48-2021 | Vaccination |
| 3 | 6 | 2-2022 | Safety evaluation and blood sampling |
| 4 | 6 | 2-2022 | Challenge |
| 5 | 10 | 6-2022 | Termination of challenge |

**Table 11: Tank allocation at vaccination:**

| **Formulation no.** | **Batch number** | **No. of fish Ah9/Tank 5 To be challenged** | **No. of fish Ah9/Tank 6 For safety & serology** |
|---|---|---|---|
| 1 | FAO-21-NED-141.20 | 35 | 25 |
| 2 | FAO-21-NED-142.6 | 35 | 25 |

**Table 12: Animals**

| **Species** | **Atlantic salmon** |
|---|---|
| Supplier | ILAB |
| Fish strain | ILAB/21/500 |
| Origin | Stofnfiskur |
| Mean weight | approx.30 gram |
| Number of fish | 585 |

**Table 13: Challenge strains**

| **Species:** | ***Aeromonas salmonicida*** |
|---|---|
| Internal strain no: | 1654_271016 |
| Challenge dose: | 300 cfu/fish |

### Inclusion and exclusion criteria, safety aspects

Only healthy fish (e.g. no deformities or noticeable injuries) was included in the study. All handling of live bacteria was done in designated areas.

**Table 14: Animal housing conditions during challenge**

| **Condition** | **Parameter** |
|---|---|
| Salinity/Conductivity | Freshwater |
| Temperature | 12 ± 2°C |
| Oxygen level | ≥ 65°/ |
| Fish density | ≤ 15 kg/m3 |
| | |
| Feeding | Ad libitum* |
| Tank size | 500 L |
| Photoperiod | 12:12 |

| | |
|---|---|
| ^{∗}Fish must be starved for 24 h prior to challenge. | |

### Challenge procedures

A total of 350 fish was anesthetized, i.m. challenged and transferred to C5/K8.

### Evaluation of results

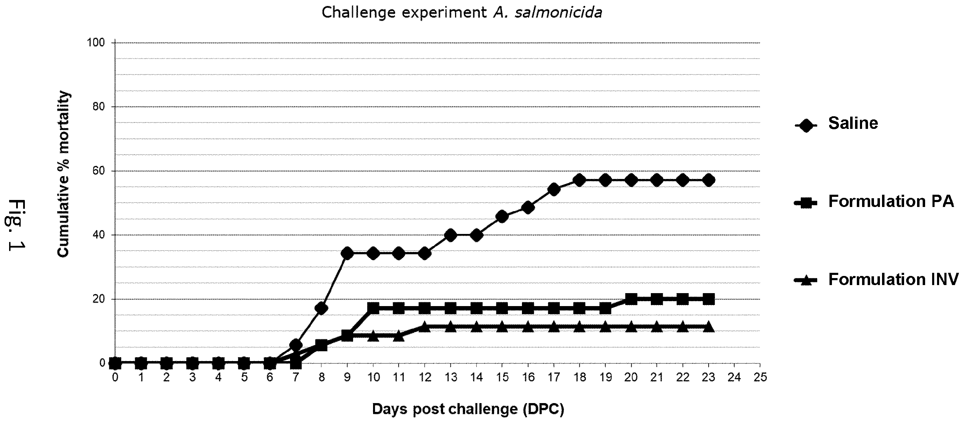

Daily mortality was recorded in each tank throughout the experiment. Moribund fish was taken out and registered as dead. Confirmation of infection as cause of disease. From 5 dead fish bacteria was recovered from the head kidney using sterile loops and plating out on blood agar plates with 0.9% and 2 % NaCl. Results are shown in Figs 1.

### Example 2: challenge trial and seroconversion test

The experiment contained the following test vaccines and controls:

**Table 15: Vaccines as used in the experiments**

| **Formulation no.** | **Vaccine** | **Batch no** | **No. of fish** | **Labelling** |
|---|---|---|---|---|
| 1 | Vaccine PA | FAO-21-NED-141.20 | 25+35+35 | RM |
| 2 | Vaccine INV v1 | FAO-21-NED-142.6 | 25+35+35 | LM |
| 3 | Vaccine INV v2 | FAO-22-NED-162.1 | 25+35+35 | RM+AF |
| 4 | Saline | TBD | 10+35+35 | AF |

The experiment has the following timeline of the *in vivo* part of the experiment:

**Table 16: Timeline of the in vivo experiment**

| **Event no.** | **Project week (PW)** | **Calendar week** | **Task** |
|---|---|---|---|
| 1 | 0 | 22 | Vaccination |
| 2 | 6 | 28 | Safety evaluation and blood sampling |
| 3 | 6 | 28 | Challenge |
| 4 | 10 | 32 | Termination of challenge |

Labelling and tank allocation was as follows:

**Table 17: Labelling and tank allocation**

| **Formulation no.** | **Batch number** | **No. of fish AH9** | | | **Challenge tank** |
|---|---|---|---|---|---|
| | | K6 | K9 | K10 | |
| 1 | FAO-21-NED-141.20 | 25 | 35 | 35 | 35 |
| 2 | FAO-21-NED-142.6 | 25 | 35 | 35 | 35 |
| 3 | FAO-22-NED-162.1 | 25 | 35 | 35 | 35 |
| 4 | TBD | 10 | 35 | 35 | 35 |

**Table 18: Tank allocation at vaccination:**

| **Formulation no.** | **Batch number** | **No. of fish Ah9/Tank 5 To be challenged** | **No. of fish Ah9/Tank 6 For safety & serology** |
|---|---|---|---|
| 1 | FAO-21-NED-141.20 | 35 | 25 |
| 2 | FAO-21-NED-142.6 | 35 | 25 |

Three tanks are needed, one for the fish to be blood sampled, and one for the fish that was moved to the challenge cell and challenged by *A.salmonicida* 6 weeks post vaccination. In addition, one tank served as backup in case something goes wrong in the first challenge experiment. If another challenge is not needed, these fish was used for an extra sampling point for blood and side effect evaluation (numbers of fish per group for second sampling limited to 25).

### Test and control articles

Test vaccines are shown in the following table (different heat treatment regimens for
*A. salmonicida.*

**Table 19: Test vaccines**

| Product name/code | Vaccine PA |
|---|---|
| Volume/dose | 0.05 ml |
| Batch number | FAO-21-NED-141.20 |
| Storage temp. | 2-8 °C |
| | |

| Product name/code | Vaccine INV v1 |
|---|---|
| Volume/dose | 0.05 ml |
| Batch number | FAO-21-NED-142.6 |
| Storage temp. | 2-8 °C |
| | |

| Product name/code | Vaccine INV v2 |
|---|---|
| Volume/dose | 0.05 ml |
| Batch number | FAO-22-NED-162.1 |
| Storage temp. | 2-8 °C |

**Table 20: Test animals**

| **Species** | **Atlantic salmon** |
|---|---|
| Supplier | ILAB |
| Fish strain | ILAB/21/501 |
| Origin | Stofnfiskur |
| Mean weight | 25-30 gram |
| Number of fish | 375 including T0 samples (n=10) |

### Inclusion and exclusion criteria

Only healthy fish (e.g. no deformities or noticeable injuries) was included in the study. Post-admission withdrawal criteria: Fish with unreadable identification was excluded from the performance evaluation.

**Table 21: Animal housing conditions**

| **Salinity/Conductivity** | **Freshwater** |
|---|---|
| Temperature | 12 ± 2°C |
| Oxygen level | ≥ 65°/ |
| Fish density | ≤ 40 kg/m³ |
| Feeding | Ad libitum* |
| Tank size | 500 L |
| Photoperiod | 12 hours |

| | |
|---|---|
| *Fish must be starved for 24 h prior to vaccination. | |

### Vaccination procedures

A total of 365 fish was taken from the reservoir tank and subjected to the following treatment:
1. Anaesthesia (Finquel vet. 120 mg/l with equal amount buffer NaHCOs)
2. Identification by maxillae clipping and adipose fin clipping as shown in table 3.
3. Vaccination using 1 ml syringes.
4. Left to recover in tank.

In addition, 10 naive fish was subjected to blood sampling.

### Sampling procedures

- On the day of vaccination, blood samples was taken from 10 unvaccinated fish, and the serum frozen with glycerol. ELISA test was done to verify absence of antibodies at the start of the experiment.
- Blood samples was taken from 25 fish from each group 6 weeks post vaccination (10 fish from saline group), and the serum processed as described in relevant SOP (doc. ID 1354).
   o Sampling of blood is performed according to SOP (doc. ID 1333).
- Weight registration:
   o Fish for serology was weighted
   o Same fish were also evaluated for side effects according to Speilberg score.

### Challenge Strain

**Table 22: Challenge strain:**

| Species | *Aeromonas salmonicida* subsp. *salmonicida* |
|---|---|
| Internal strain no._batch no. | 1654_271016 |
| Challenge dose | approx. 200 cfu/fish |

Challenge was performed by intramuscular injection of 0.05 ml solution. Stock solution from vial was diluted in 0.9 % NaCl.

### Evaluation of results

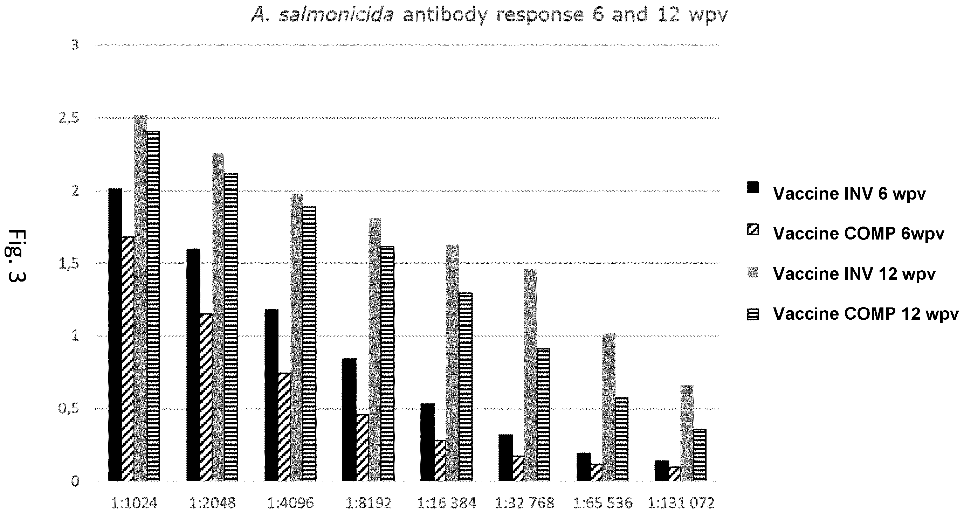

Blood samples was analysed by ELISA against *A.salmonicida*, *M.viscosa* and *Y.ruckeri.* Side effect evaluation according to the Speilberg scored was plotted and analysed in excel. For results see *Figs. 3* *A* and B.

### Example 3

**Table 23: Test vaccines and controls**

| **Formulation no.** | **Vaccine** | **Batch no** | **No. of fish** | **Labelling** |
|---|---|---|---|---|
| 1 | Vaccine COMP | 613879 | 25+35+35 | RM |
| 2 | Vaccine INV v1 | FAO-21-NED-142.6 | 25+35+35 | LM |
| 3 | Vaccine INV v1 | FAO-22-NED-162.1 | 25+35+35 | RM+AF |
| 4 | Saline | 120422 | 10+35+35 | AF |

The experiment has the following timeline of the *in vivo* part of the experiment:

**Table 24: Timeline of the experiment.**

| **Event no.** | **Project week (PW)** | **Calendar week** | **Task** |
|---|---|---|---|
| 1 | 0 | 38 | Vaccination |
| 2 | 6 | 44 | Safety evaluation and blood sampling |
| 3 | 6 | 44 | Challenge |
| 4 | 10 | 45 | Termination of challenge |

**Table 25: Labelling and tank allocation**

| **Formulation no.** | **Batch number** | **No. of fish AH9** | | | **Challenge tank** |
|---|---|---|---|---|---|
| | | K9 | K8 | K7 | |
| 1 | 613879 | 25 | 35 | 35 | 35 |
| 2 | FAO-21-NED-142.6 | 25 | 35 | 35 | 35 |
| 3 | FAO-22-NED-162.1 | 25 | 35 | 35 | 35 |
| 4 | 120422 | 10 | 35 | 35 | 35 |

Three tanks are needed one for the fish to be blood sampled, and one for the fish that was moved to the challenge cell and challenged by *A.salmonicida* approximately 6 weeks post vaccination. In addition, one tank served as backup in case something goes wrong in the first challenge experiment. If another challenge is not needed, these fish was used for an extra sampling point for blood and side effect evaluation (numbers of fish per group for second sampling limited to 25).

### Test and control articles

Test vaccines are shown in the following table (different heat treatment regimens for
*A. salmonicida.*

**Table 26: Test vaccines**

| Product name/code | Vaccine COMP |
|---|---|
| Supplier | n/a |
| Volume/dose | 0.05 ml |
| Batch number | n/a |
| Storage temp. | 2-8 °C |
| | |

| Product name/code | Vaccine INV v1 |
|---|---|
| Supplier | Vaxxinova Netherland |
| Volume/dose | 0.05 ml |
| Batch number | FAO-21-NED-142.6 |
| Storage temp. | 2-8 °C |
| | |
| Supplier | Vaxxinova Netherland |
| Volume/dose | 0.05 ml |
| Batch number | FAO-22-NED-162.1 |
| Storage temp. | 2-8 °C |
| | |

| Product name/code | 0.9 % saline |
|---|---|
| Supplier | Vaxxinova Norway |
| Volume/dose | 0.05 ml |
| Batch number | 120422 |
| Storage temp. | 2-8 °C |

**Table 27: Test animals**

| **Species** | **Atlantic salmon** |
|---|---|
| Supplier | ILAB |
| Fish strain | ILAB/21/503 |
| Origin | Stofnfiskur |
| Mean weight | 35 gram |
| Number of fish | 375 including TO samples (n=10) |

### Inclusion and exclusion criteria

Only healthy fish (e.g. no deformities or noticeable injuries) was included in the study. Post-admission withdrawal criteria: Fish with unreadable identification was excluded from the performance evaluation.

Dead or dying animals was excluded from the performance evaluation if cause of death is determined to be unrelated to vaccination, e.g., wrongful handling, infectious disease or other technical errors that may occur.

**Table 28: Animal housing conditions**

| **Salinity/Conductivity** | **Freshwater** |
|---|---|
| Temperature | 12 ± 2°C |
| Oxygen level | ≥ 65°/ |
| Fish density | ≤ 40 kg/m³ |
| Feeding | Ad libitum* |
| Tank size | 500 L |
| Photoperiod | 12 hours |

| | |
|---|---|
| *Fish must be starved for 24 h prior to vaccination. | |

### Vaccination procedures

A total of 365 fish was taken from the reservoir tank and subjected to the following treatment:
1. Anaesthesia (Finquel vet. 120 mg/l with equal amount buffer NaHCOs)
2. Identification by maxillae clipping and adipose fin clipping as shown in table 3.
3. Vaccination using 1 ml syringes.
4. Left to recover in tank.
In addition, 10 naive fish was subjected to blood sampling.

### Sampling procedures

- On the day of vaccination, blood samples was taken from 10 unvaccinated fish, and the serum frozen with glycerol. ELISA test was done to verify absence of antibodies at the start of the experiment.
- Blood samples was taken from 25 fish from each group 6 weeks post vaccination (10 fish from saline group), and the serum processed as described in relevant SOP (doc. ID 1354).
   ∘ Sampling of blood is performed according to SOP (doc. ID 1333).
- Weight registration:
   o Fish for serology was weighted
   o Same fish were also evaluated for side effects according to the modified Speilberg score.

### Challenge Strain

**Table 29: Challenge strain**

| Species | *Aeromonas salmonicida* subsp. *salmonicida* |
|---|---|
| Internal strain no._batch no. | 1654_27101. Nye smitteampuller 19.09.2023. |
| Challenge dose | selected from pre-challenge test |

Challenge was performed by intramuscular injection of 0.05 ml solution. Stock solution from vial was diluted in 0.9 % NaCl.

### Evaluation of results

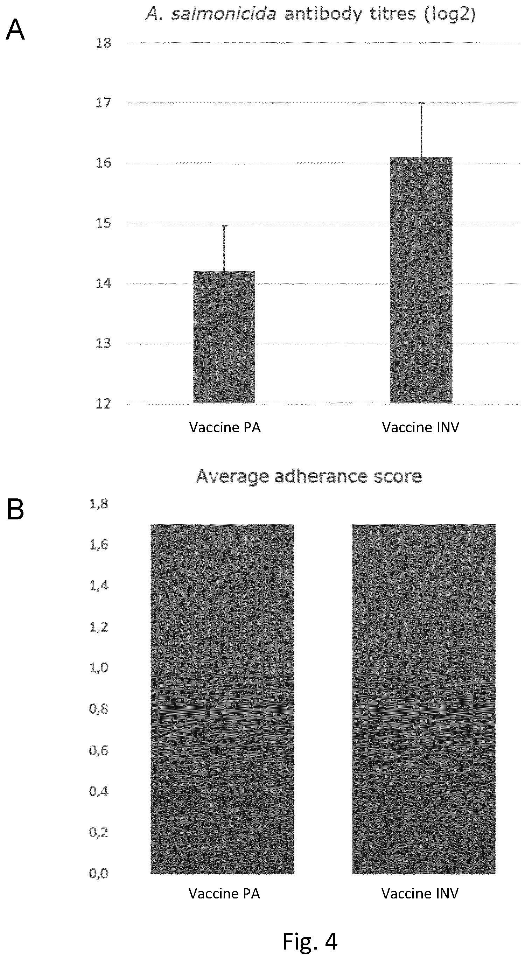

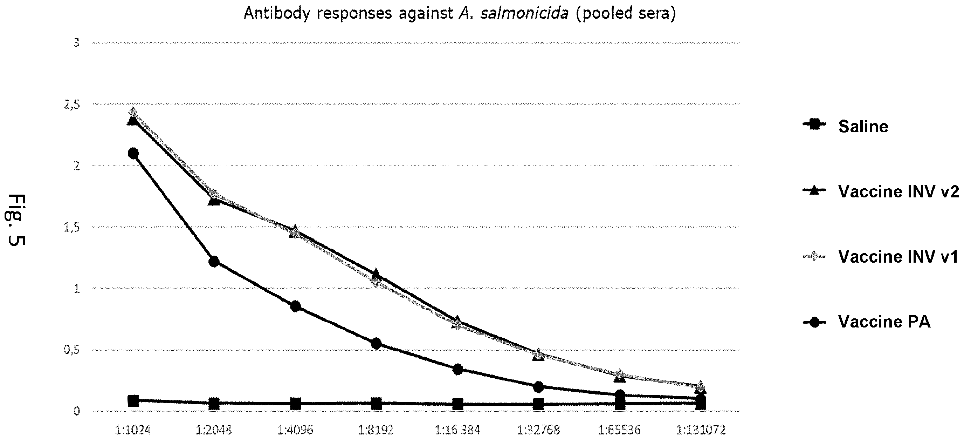

Blood samples was analysed by ELISA *against A.salmonicida*, *M.viscosa* and *Y.ruckeri.* Side effect evaluation according to the Speilberg scored was plotted and analysed in excel. For results see Figs. 4A and 5.

### References

The content of the prior art documents referred to herein is incorporated by reference. This refers, particularly, for prior art documents that disclose standard or routine methods. In that case, the incorporation by reference has mainly the purpose to provide sufficient enabling disclosure, and avoid lengthy repetitions.
- Woodard LF, Jasman RL. Stable oil-in-water emulsions: preparation and use as vaccine vehicles for lipophilic adjuvants. Vaccine. 1985 Jun;3(2): 137-44
- Midtlyng PJ (1996) A field study on intraperitoneal vaccination of Atlantic salmon (Salmo salar L.) against furunculosis. Fish Shellfish Immunol 6:553-565)

## Claims

1. A vaccine formulation comprising at least one inactivated virus or bacterium, or an immunogenic fragment thereof, the formulation further comprising one or more surfactants, wherein at least one surfactant does not comprise an ester bond.

2. The vaccine formulation according to claim 1, wherein the surfactant is a Polyalkylenglycolether or a Polyoxyethylenoleylether.

3. The vaccine formulation according to claim 1, wherein the surfactant that does not comprise an ester bond has
a) a molecular weight of ≥ 600 g/mol, and/or
b) a Hydrophilic-Lipophilic Balance (HLB) of ≥ 8.

4. The vaccine formulation according to any one of the aforementioned claims, wherein the surfactant is Brij^{®}O20

5. The vaccine formulation according to any one of the aforementioned claims, wherein the formulation further comprises at least one hydrophobic ingredient selected from the group consisting of
• lipid
• natural oil,
• partially hydrogenated natural oil,
• mineral oil,
• branched hydrocarbon and/or
• unbranched hydrocarbon

6. The vaccine formulation according to any one of the aforementioned claims, wherein the vaccine is polyvalent and comprises two or more inactivated virus or bacteria, or immunogenic fragments thereof.

7. The vaccine formulation according to any one of the aforementioned claims, wherein the virus or bacteria have been inactivated by application of at least one of
• formaldehyde
• heat,
• radiation
• ethyleneimine and/or
• beta-propiolactone (BPL)

8. The vaccine formulation according to any one of the aforementioned claims, wherein at least one bacterium in the formulation is a bacterium that produces, secretes and/or releases after lysis a lipase, optionally a heat stable lipase.

9. The vaccine formulation according to any one of the aforementioned claims, wherein the formulation comprises one or more further surfactants.

10. The vaccine formulation according to claim 9, wherein, one such further surfactant is a surfactant that comprises an ester bond., optionally wherein, one such further surfactant that comprises an ester bond is Sorbitan monooleate (Span 80^{®}).

11. The vaccine formulation according to claim 10, wherein one such further surfactant that comprises an ester bond has a Hydrophilic-Lipophilic Balance (HLB) of ≤ 9.

12. A vaccine comprising the vaccine formulation according to any one of the aforementioned claims.

13. Use of the vaccine formulation according to any one of the aforementioned claims for the manufacture of a vaccine.

14. The formulation according any one of the aforementioned claims (for the manufacture of a vaccine) for the treatment of an animal or human.

15. The formulation or vaccine according to anyone of the aforementioned claims, wherein the animal is at least one selected from the group consisting of bird, mammals including ruminants, non-ruminants, and ungulates, and fish.
